# EUROPEAN PATENT SPECIFICATION

(11) **EP 2 782 446 B1**
(45) Date of publication and mention of the grant of the patent: **24.02.2016**
(21) Application number: 12781308.7
(22) Date of filing: 01.11.2012
(51) Int. Cl.: A01N 25/04, A01N 31/04, A01N 31/08, A01N 31/16, A01N 33/12, A01P 1/00, A61K 8/34, A61K 8/49, A61Q 11/00, A61Q 17/00

(54) **TRANSPARENT ANTIMICROBIAL MICROEMULSION COMPOSITION**
TRANSPARENTE ANTIMIKROBIELLE MIKROEMULSIONSZUSAMMENSETZUNG
COMPOSITION DE MICROÉMULSION ANTIMICROBIENNE TRANSPARENTE

(30) Priority: 25.11.2011 IN MM33292011; 12.01.2012 EP 12150937
(43) Date of publication of application: 01.10.2014
(73) Proprietor: Unilever N.V., 3013 AL Rotterdam (NL); Unilever PLC, London, Greater London EC4Y 0DY (GB)
(72) Inventor: MADHAVAN, Uma, Whitefield Bangalore 560 066 (IN); ROUT, Deeleep Kumar, Whitefield Bangalore 560 066 (IN); SAJI, Maya Treesa, Whitefield Bangalore 560 066 (IN); SINHA, Ritesh Kumar, Whitefield Bangalore 560 066 (IN)
(74) Representative: van den Brom, Coenraad Richard
(86) International application number: PCT/EP2012/071650
(87) International publication number: WO 2013/075921

(56) References cited:
- EP-A1- 2 018 869
- WO-A1-2011/036048
- CN-A- 101 036 459
- CN-A- 101 601 382
- CN-A- 101 874 531
- US-A- 6 114 298
- US-A1- 2004 209 795
- US-A1- 2006 045 914

## Description

### FIELD OF INVENTION

The present invention relates to liquid antimicrobial composition, particularly to liquid antimicrobial composition in a stable micro-emulsion form.

The invention has been developed primarily for use in personal cleaning, oral care, hard and soft surface cleaning and will be described hereinafter with reference to these applications.

### BACKGROUND AND RELATED ART

Any discussion of the prior art throughout the specification should in no way be considered as an admission that such prior art is widely known or forms part of the common general knowledge in the field.

It is known to add antimicrobial agents to aqueous cleaning composition for use on hard surfaces and soft surfaces. Addition of antimicrobial agents to these aqueous compositions provides better cleaning and disinfection action. Essential oils are natural anti-microbial agents. When used in right combination essential oils provide high level of disinfection and also give the consumers satisfaction of having used natural products, which they believe have fewer side effects on them and which impact the environment minimally.

Such natural actives like essential oils are insoluble in water and have to be formulated in water with the aid of emulsifiers which lead to a composition that is opaque or turbid and kinetically unstable. Providing such a composition in a transparent or translucent form is highly appealing to the consumer.

One way of including oils in water, while ensuring transparency is to formulate them in a micro-emulsion form. Some of the agents used for solubilising essential oils in aqueous composition include lower molecular weight alcohol or surfactants. It has been known in the art to provide micro-emulsion composition having essential oils with either or both of surfactants and alcohols.

CN101036459A (Jiangsu Academy of Agricultural Sciences, 2007) discloses a micro-emulsion composition and the preparation method thereof providing high efficiency, low toxicity, low production cost, simple preparation process, good safety in production, storage and transportation, and use process, and good environmental protection performance. The micro-emulsion disclosed in the invention includes eugenol, solvent, emulsifying agent, synergic aid, anti-freezing agent, and water. US6,114,298 A (The Procter and Gamble Company, 2000) discloses a micro-emulsion composition and its use for disinfecting surface. This application discloses a disinfecting composition having a surfactant, a bleach preferably a peroxygen bleach, an essential oil or an active thereof and an aqueous phase formulated in the form of a micro-emulsion comprising droplets of essential oil or an active thereof dispersed in said aqueous phase, and having a particle size of less than 100 nanometers provides an improved disinfecting performance. The invention discloses composition having upto 15 wt% hydroxylated solvents such as glycol ethers and/or derivatives thereof, polyols, alkoxylated aliphatic or aromatic alcohols, aliphatic or aromatic alcohols, glycols or mixtures for promoting the formation of the micro-emulsions in addition to the surfactants.

Our co-pending application in India 3320/MUM/2010 (Unilever, 2010) discloses a liquid antimicrobial composition that has a clear transparent / translucent appearance and becomes turbid on dilution with water and its use as a disinfecting composition. The invention discloses a composition having antimicrobial active selected from the class comprising terpenes, essential oils, or cationic oils; surfactant; organic solvent selected from linear or branched alcohol and a selective clouding agent.

Another co-pending application in India 3321/MUM/2010 (Unilever, 2010) discloses a transparent/translucent liquid antimicrobial mouthwash composition that is substantially free of low molecular weight alcohol. The invention discloses a composition having antimicrobial active selected from the class comprising terpenes, essential oils, cationic oils, fatty acids, fatty acid methyl esters, organic per-acids, or mono, di and triglycerides of fatty acids; surfactant; electrolyte and water.

WO 2011/036048 discloses a liquid antimicrobial composition comprising eugenol, thymol, terpineol, a surfactant and water. Particularly preferred is the use of a cationic surfactant, such as benzalkonium chloride.

It has been unexpectedly found by the present inventors that increasing the amount of lower molecular weight alcohol or electrolytes does not solubilise higher levels of essential oils added to form a micro-emulsion composition. It is thus a challenge to formulate an aqueous antimicrobial composition in a micro-emulsion format that includes high levels of essential oils while still having a transparent/ translucent appearance.

There is a need in the art for achieving stable micro-emulsion oil-in-water antimicrobial compositions with higher levels of essential oil active, and still have good stability.

We have observed that when polyphenols with at least 50% catechins extracted from *Camellia sinensis* and comprising at least 25% epigallocatechin gallate is added to an aqueous antimicrobial composition comprising essential oil actives selected from thymol, terpineol, eugenol and mixtures thereof

it provides for a stable micro-emulsion having increased amounts of essential oil actives. It was surprisingly found that polyphenols with at least 50% catechins cause a significant increase in stability of aqueous antimicrobial micro emulsion compositions containing high levels of essential oil actives. Such an activity of polyphenols was not known. It also allows for significant reduction in the level of electrolytes or removal of electrolyte from the composition, while still being highly stable.

### OBJECT OF THE INVENTION

It is an object of the present invention to overcome or ameliorate at least one of the disadvantages of the prior art.

Other objects of the present invention will become apparent to those skilled in the art by reference to the specification.

### SUMMARY OF THE INVENTION

According to the first aspect, disclosed is an oil-in-water antimicrobial micro-emulsion composition comprising:
(i) 0.1 to 10 wt% surfactant;
(ii) 0.05 to 10 wt% essential oil active comprising thymol, terpineol, eugenol or mixtures thereof;
(iii) 0.1 to 5 wt% polyphenol; and,
(iv) 80 to 98 wt% water;
wherein said polyphenol comprises at least 50% of catechins and wherein said catechins comprise at least 25% epigallocatechin gallate;
and wherein the polyphenol is extracted from *Camellia sinensis.*

According to a second aspect, disclosed is a non-therapeutic cosmetic method of disinfecting a surface comprising the steps of:
(i) applying a composition of the first aspect of the invention to a desired surface; and,
(ii) removing the composition from said surface after a predetermined time period. According to another aspect, the present invention also provides a composition according to the invention for use in disinfecting external surfaces of human or animal body, disinfecting soft and porous substrates like fabric or disinfecting hard surfaces. For a more complete understanding of the above and other features and advantages of the invention, reference should be made to the following detailed description of preferred embodiments.

### DETAILED DESCRIPTION OF THE INVENTION

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention.

Similarly, all percentages are weight/weight percentages unless otherwise indicated. Numerical ranges expressed in the format "from x to y" are understood to include x and y. When for a specific feature multiple preferred ranges are described in the format "from x to y", it is understood that all ranges combining the different endpoints are also contemplated.

Disclosed antimicrobial composition includes an oil-in-water micro-emulsion having a surfactant, essential oil active comprising thymol, terpineol, eugenol or mixtures thereof, polyphenol comprising at least 50% catechins wherein said catechins comprise at least 25% epigallocatechin gallate and wherein the polyphenol is extracted from *Camellia sinensis* and water.

### Oil-in-water micro-emulsion:

Disclosed antimicrobial composition is an oil-in-water micro-emulsion having 80 to 98 wt% water.

Micro-emulsions have a transparent or translucent appearance, as opposed to an opaque or milk like appearance typically associated with emulsions.

The micro-emulsions herein are also physically stable. By "physically stable" it is meant herein that the micro-emulsions do not show phase separation upon prolonged storage, i.e., the droplets having essential oils/actives remain dispersed in the aqueous phase.

Micro-emulsions may be defined as an emulsion having ultra small, example in the order of several nanometres to about 200 nm diameters of the dispersed phase particles. Micro-emulsions are described in (Evans, D. F.; Wennerstrom, H. (Eds.); 'The Colloidal Domain', Wiley-VCH, New York, (1999)). Micro-emulsions are clear and thermodynamically stable and, therefore, are for the man skilled in the art different from ordinary emulsions the latter being thermodynamically unstable and generally turbid. Particle size of dispersed phase particles is preferably less than 100 nanometres, more preferably less than 90 nm, and still more preferably less than 50 nm.

Preferred composition has a pH of 4 to 7. Disclosed antimicrobial composition may preferably include a buffer. Preferred buffer system include citric acid and sodium citrate.

### Surfactant

Disclosed antimicrobial composition includes 0.1 to 10 wt% surfactant. Preferred composition include 0.1 to 8 wt% surfactant. More preferred composition include 0.1 to 5 wt% surfactant.

In general, the surfactants may be chosen from the surfactants described in well known textbooks like "Surface Active Agents" Vol. 1, by Schwartz & Perry, Interscience 1949, Vol. 2 by Schwartz, Perry & Berch, Interscience 1958, or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used.

The surfactant is preferably of the cationic, anionic, or zwitterionic class, most preferably of the cationic class. When anionic surfactant is present it is preferably chosen from alkali or alkaline earth metal salts of alkyl sulphonic acid, fatty acid, or alkyl ether sulphate. When zwitterionic surfactant is present it is preferably chosen from betaines, sulphobetains, hydroxyl sulphobentains, amino carboxylates; when a cationic surfactant is present it is preferably benzalkonium chloride, alkyl pyridinium chloride or quaternary ammonium gemini surfactants. Preferably cationic surfactant is benzalkonium chloride or cetyl trimethyl ammonium bromide.

### Essential oil active

Disclosed antimicrobial composition includes 0.05 to 10 wt% essential oil active including thymol, terpineol, eugenol or mixtures thereof. Preferred composition includes 0.1 to 8 wt% of essential oil active. More preferred compositions include 0.3 to 8 wt% essential oil active. Further preferred compositions include 0.3 to 6 wt% essential oil active, and optimal compositions include 0.4 to 5 wt% of essential oil active.

Certain other essential oil active which may optionally also be used herein includes, amyl salicylate, carvacrol, cymene, e.g. p-cymene, dihydroeugenol, eugenol, hexyl eugenol, hexyl salicylate, isoeugenol, methyl eugenol, methyl isoeugenol, methyl salicylate, tert butyl cresol, thymol, and vanillin.

Certain other non-aromatic essential oils of terpenoid compounds which may optionally also be used herein include cedrene, cineole, citral (including geranial and neral), citronellal, citronellol, eucalyptol (also known as 1,8 cineole) paradihydrolinalool, dihydromyrcenol (DH myrcenol), farnesol, geraniol, hexyl cinnamaldehyde, hydroxycitronallol, hydroxycitronellal, isocitral, limonene, d-limonene, linalool, longifolene, menthol, nerol, nerolidiol, pinene, e.g. α-pinene, phellendrene, terpinene, e.g. α-terpinene and γ-terpinene, terpineol, e.g. γ-terpineol and terpin-4-ol, and tetrahydromyrcenol (THM).

Preferred compositions have 0.01 to 5 wt% terpineol more preferably 0.3 to 2wt% terpineol. It is highly preferred that the composition has 0.03 to 2 wt% and further more preferably 0.03 to 0.5 wt% terpineol. Preferred compositions have 0.1 to 2 wt% thymol more preferably 0.1 to 0.9 wt%. It is highly preferred that the composition has 0.01 to 2wt% and more preferably 0.01 to 0.9wt% thymol. Preferred composition has 0.01 to 2 wt% eugenol more preferably 0.02 to 0.2 wt% eugenol. A preferred aspect of the composition includes thymol and terpineol and a more preferred aspect includes thymol, terpineol and eugenol.

### Polyphenol

Disclosed antimicrobial composition includes 0.1 to 5 wt% polyphenol. Polyphenols are a group of chemical substances found in plants, characterized by the presence of more than one phenol unit or building block per molecule. Polyphenols are generally divided into hydrolyzable tannins (gallic acid esters of sugars) and phenylpropanoids, such as lignins (secoisolariciresinol diglycoside), flavonoids, and condensed tannins.

The largest class and best studied polyphenols are the flavonoids, which include several thousand compounds, among them the flavonols (Quercetin, Gingerol, Kaempferol, Myricetin, Resveratrol, Rutin), flavones (Apigenin, Luteolin), catechins (Epicatechin, Catechin Gallates, Theaflavin), flavanones (Hesperiden, Naringenin, Silibenin, Eriodictyol), anthocyanidins (Pelargonidin, Peonidin, Cyanidin, Delphinidin, Malvidin) isoflavonoids (Daidzein, Genistein, Glycitein) and coumestans (Coumestrol). Preferred compositions include 0.2 to 4 wt% polyphenol. More preferred compositions include 0.2 to 4 wt% polyphenol. Further preferred compositions include 0.25 to 3 wt% polyphenol.

Disclosed Polyphenol includes at least 50% catechins. Preferred composition has at least 65% of catechins, and optimal composition includes 100% catechins. The catechins include at_least 25% epigallocatechin gallate. The polyphenol is extracted from green tea (Camellia sinensis).

Preferred commercially available polyphenol halving at least 50% catechins includes Sunphenon 90 LB and Sunphenon 80SK (Taiyo Kagaku Co., Ltd).

### Electrolytes

The antimicrobial composition may preferably include electrolytes. The electrolytes may be selected from a chloride, sulphate, acetate, or carbonate of an alkali, alkaline earth or a transition metal. Of these, multivalent electrolytes are especially preferred. More useful electrolytes include chloride, sulphate or acetate of an alkaline earth metal. Examples of electrolytes useful in including in the disclosed composition are calcium chloride, sodium chloride, magnesium chloride, zinc sulphate, aluminium chloride or zinc acetate. Electrolyte is preferably present in 0.5 to 2 wt% more preferably 0.5 to 1.5 wt% of the composition.

It is preferred that the composition of the invention includes less than 1%, preferably less than 0.5%, more preferably less than 0.1 % C1 - C3 alcohol and in optimal compositions it is absent.

### Method of disinfecting a surface:

According to a second aspect, disclosed is a non-therapeutic method of disinfecting a surface, the method including the steps of:
a. applying a composition of the first aspect to the surface; and, optionally,
b. rinsing the surface with a suitable solvent, or wiping the surface after a predetermined time period.

The composition is preferably used for disinfecting external surfaces of human or animal body, for disinfecting soft and porous substrates like fabric or for disinfecting hard surfaces.

In use, preferably, the composition is contacted with the surface e.g. hand, face, body, oral cavity, or any hard surface e.g. a utensil.

It is then rinsed with sufficient amounts of water after a pre-determined period of time to remove any visible or sensory residue of the composition. Alternatively an alcohol wipe or a water/alcohol impregnated wipe may be used to wipe the surface to be visibly free of the anti-microbial composition. Preferably the step of rinsing is carried out for less than 5 minutes, more preferably less than 2 minutes, further more preferably less than a minute and in many cases less than 15 seconds after the step of applying the composition on the substrate.

According to yet another aspect, the present invention provides for a composition according to the invention for use in disinfecting external surfaces of human or animal body, disinfecting soft and porous substrates like fabric or disinfecting hard surfaces. The use is non-therapeutic.

### Formats

Antimicrobial compositions in liquid micro-emulsion form are useful for skin cleansing, in particular as hand-wash, body-wash and face-wash or useful as a mouthwash. The liquid antimicrobial compositions may be used for disinfecting hard surfaces, fabrics, nappies and for cut, scratched or bruised skin. Some liquid compositions may be sold as concentrated antimicrobial compositions which are often used in diluted form at a dilution ratio of 1:1 to 1:100, more preferably 1:10 to 1:50.

Disclosed antimicrobial compositions may be packaged in manually operated spray dispensing containers, preferably in a trigger spray dispenser or in a pump spray dispenser. Spray-type dispensers allow for uniformly applying to a relatively large area of a surface to be disinfected, and are particularly suitable to treat vertical surfaces. Spray dispensers may also be so designed to help atomize the disclosed antimicrobial composition. The antimicrobial composition of the present invention may also be executed in the form of wipes. By "wipes" it is meant herein disposable towels incorporating a micro-emulsion according to the present invention. For example the disclosed antimicrobial composition may be impregnated/wetted in disposable paper towels.

The invention will now be demonstrated with examples.

### EXAMPLES

### Preparation of comparative and preferred aqueous antimicrobial composition

Surfactant system according to the formulation given in Table 1 and 2 was dissolved in 90 ml of water. To this surfactant solution essential oil actives were added drop-wise and stirred. Addition of the essential oil active was continued until a turbid solution was obtained. In the next step, 0.1 to 0.2 grams of a solubilising agent was added and this process of addition of solubilising agent was continued until a transparent solution was obtained. The resultant transparent solution was then made up to 100% by addition of water.

### Examples 1:

### Efficacy of various mouthwash compositions against S. mutans in a 30 seconds contact test in a suspension

Streptococcus mutans (S. mutans) is a Gram-positive, facultative anaerobic bacterium commonly found in the human oral cavity. S. mutans is the leading cause of dental caries (tooth decay) worldwide and is considered to be the most cariogenic of all of the oral streptococci. S.mutans, sticks to the surface of teeth and subsists on a diverse group of carbohydrates. While metabolising sugar and other energy sources, the microbe produces acid that causes cavities in teeth.

The compositions of Table-1 and Table-2 were used to test the efficacy as a antibacterial mouth wash composition against S. mutans in a 30 second contact test in suspension, using the following procedure.

Sub cultures of S.mutans in a BHI broth ((Brain Heart Infusion Broth- Difco- 37 grams per liter) were taken and allowed to grow in CO₂ incubator (15% CO₂) at 37°C for 15 hours. The culture was adjusted to a cell density of 10⁸ cfu/ml (optical density ~ 0.3 at 620nm). 1 ml of this culture were added to sterile containers having 9ml of the comparative composition or the compositions according to the present invention and mixed thoroughly. After a 30 second contact time with the culture, they were neutralized in D/E broth by transferring 1 ml of it into 9ml of D/E broth.

After serial dilution they were plated in BHI Agar (Difco- 52gpl). The plates were incubated in CO₂ incubator. The residual colonies were counted after 48 hours incubation.

The log reduction of the bacteria was measured and the data is summarized in Table-1 and Table-2.

### Example 2: Stability of micro-emulsion compositions

Aqueous antimicrobial compositions having the ingredients as provided in Table 1 were formulated following the preparation protocol as disclosed earlier. Comparative examples had Zinc sulphate·7H₂O as a solubilising agent. Compositions according to the present invention included Sunphenon 80SK (Taiyo Kagaku Co., Ltd) as the solubilising agent. (Example 1). The compositions have been described in Table 1.

The turbidity of various comparative and experiment compositions was measured using a turbidometer which is also described in Table 1.

**Table 1**

| Ingredients (%w/w) | Example a | Example b | Example 1 |
|---|---|---|---|
| Thymol^{#} | 0.12 | 0.83 | 0.83 |
| Terpineol^{#} | 0.25 | 1.64 | 1.64 |
| Eugenol^{#} | 0.02 | 0.16 | 0.16 |
| Peppermint oil^{#} | - | 0.26 | 0.26 |
| Sodium lauryl ether sulfate | 0.4 | 2.63 | 2.63 |
| Zinc sulphate.7H₂O | 0.12 | 0.8 | 0.8 |
| Sunphenon 80SK* | - | - | 3 |
| Water | Balance to 100 | Balance to 100 | Balance to 100 |
| Appearance | Transparent micro-emulsion | Turbid | Transparent micro-emulsion |
| Turbidity (in NTU units) | 90 | 1000 | 10 |
| Log reduction | 7.0 | 7.0 | 7.0 |

| | | | |
|---|---|---|---|
| * Green tea extract (polyphenols) enriched in catechins and having 60% catechins. # Essential oil active. | | | |

It is evident from the data in Table 1 that antimicrobial composition according to the present invention, Example-1, provides a stable transparent micro-emulsion when higher levels of essential oil active is incorporated while the comparative Example-b does not form a stable micro-emulsion composition at higher levels of essential oil actives.

### Example 3: Aqueous antimicrobial composition with an anionic surfactant system

Aqueous antimicrobial compositions having linear sodium alkylbenzene sulfonate as the anionic surfactant system was prepared according to the present invention and was compared with comparative aqueous compositions having ethanol as the solubilising agent.

Turbidity of the resultant micro-emulsion compositions were measured using a turbidometer. The compositions and turbidity data are provided in Table 2.

**Table 2**

| Ingredients | Example c | Example 2 |
|---|---|---|
| Sodium linear alkylbenzene sulfonate | 5.68 | 5.68 |
| Essential oil active* | 0.5 | 0.5 |
| Sunphenon 80SK# | - | 2 |
| Ethanol | 4 | - |
| Water | To 100 | To 100 |
| Appearance | Transparent micro-emulsion | Transparent micro-emulsion |
| Turbidity (in NTU units) | 15 | 7 |
| Log reduction | 7.0 | 7.0 |

| | | |
|---|---|---|
| * a mixture of thymol ( 0.16 %), terpineol ( 0.31%) and eugenol ( 0.03%). # Green tea extract enriched in catechins and having 60% catechins. | | |

It is clear from the data in Table 2 that aqueous antimicrobial compositions according to the present invention, Example 2, provides a stable micro-emulsion at lower levels of solubilising agent i.e; polyphenols with at least 50% catechins according to claim 1 as compared to high levels of solubilising agent, ethanol required for obtaining stable micro-emulsion in the Comparative Example-c. Alcohol especially ethanol is considered as a harsh chemical and socially unacceptable in many societies even for oral or topical application.

### Example 4: Aqueous antimicrobial composition with a cationic surfactant system

Aqueous antimicrobial compositions having Benzalkonium chloride or CTAB as a cationic surfactant system was prepared according to the present invention and was compared with comparative aqueous compositions having ethanol as a solubilising agent. Turbidity of the resultant micro-emulsion compositions were measured using a turbidometer. The compositions and turbidity data are provided in Table 3.

**Table 3**

| Ingredient | Example d | Example e | Example 3 | Example 4 |
|---|---|---|---|---|
| Benzalkonium chloride | 4 | - | 4 | - |
| Cetyl trimethyl ammonium bromide | - | 3 | - | 3 |
| Essential oil active* | 1.5 | 1.5 | 1.5 | 1.5 |
| Sunphenon 80SK# | - | - | 0.3 | 1 |
| Ethanol | 7 | 4 | - | - |
| Water | 87.5 | 94 | 94.2 | 94 |
| Appearance | Transparent micro-emulsion | Transparent micro-emulsion | Transparent micro-emulsion | Transparent micro-emulsion |
| Turbidity (in NTU units) | 10 | 15 | 2 | 5 |
| Log reduction | 7.0 | 7.0 | 7.0 | 7.0 |

| | | | | |
|---|---|---|---|---|
| * a mixture of thymol (0.47%), terpineol (0.94%) and eugenol (0.09 %). # Green tea extract (polyphenols) enriched in catechins and having 60% catechins. | | | | |

It is evident from Table 3 that aqueous antimicrobial compositions having a cationic surfactant provides stable micro-emulsion in the preferred compositions (Example 3 and 4) at relatively low levels of solubilising agent (i.e; polyphenols with at least 60% catechins) as compared to high levels of solubilising agent (i.e; ethanol) required for obtaining stable micro-emulsion in the comparative examples (Example-d and Example-e).

It will be appreciated that the illustrated examples provide for a composition and methods for disinfecting surfaces. Disclosed compositions having high levels of antimicrobial essential oil actives provide a stable transparent micro-emulsion aqueous composition. The illustrated examples also explain that high essential oil active composition having polyphenols with atleast 50% catechins provides more stable micro-emulsion composition as compared to compositions with alcohols or electrolytes as solubilising agents.

## Claims

1. An oil-in-water antimicrobial micro-emulsion composition comprising:
(i) 0.1 to 10 wt% surfactant;
(ii) 0.05 to 10 wt% essential oil active comprising thymol, terpineol, eugenol or mixtures thereof;
(iii) 0.1 to 5 wt% polyphenol; and,
(iv) 80 to 98 wt% water;
wherein said polyphenol comprises at least 50% catechins and wherein said catechins comprise at least 25% epigallocatechin gallate;
and wherein the polyphenol is extracted from *Camellia sinensis.*

2. A composition as claimed in claim 1 comprising 0.01 to 5 wt% terpineol.

3. A composition as claimed in claim 1 or 2 comprising 0.1 to 2 wt% thymol.

4. A composition as claimed in any one of claims 1 to 3 comprising 0.01 to 2 wt% eugenol.

5. A composition as claimed in any one of the preceding claims wherein said surfactant is a cationic, anionic or zwitterionic surfactant.

6. A composition as claimed in claim 5 wherein said surfactant is cationic.

7. A composition as claimed in claim 6 wherein said cationic surfactant is benzalkonium chloride or cetyl trimethyl ammonium bromide.

8. A composition as claimed in any one of the preceding claims having a pH of 4 to 7.

9. A composition as claimed in any one of the preceding claims comprising 0.5 to 2 wt% electrolytes.

10. A composition as claimed in any one of the preceding claims for use in disinfecting external surfaces of human or animal body, for disinfecting soft and porous substrates like fabric or for disinfecting hard surfaces.

11. A non-therapeutic method of disinfecting a surface comprising the steps of:
(i) applying a composition as claimed in any one of the preceding claims 1 to 10 to a desired surface; and,
(ii) removing the composition from said surface after a predetermined time period.

12. A non-therapeutic method as claimed in claim 11 wherein said composition is diluted with water in a ratio of 1:1 to 1:100 before applying on said surface.

## Patentansprüche

1. Antimikrobielle Öl-in-Wasser-Mikroemulsionszusammensetzung, umfassend:
(i) 0,1 bis 10 Gew.-% Tensid,
(ii) 0,05 bis 10 Gew.-% eines essentiellen Ölwirkstoffs, umfassend Thymol, Terpineol, Eugenol oder Mischungen davon,
(iii) 0,1 bis 5 Gew.-% Polyphenol und
(iv) 80 bis 98 Gew.-% Wasser,
wobei das Polyphenol mindestens 50% Catechine umfasst und wobei die Catechine mindestens 25% Epigallocatechingallat umfassen und
wobei das Polyphenol aus Camellia sinensis extrahiert ist.

2. Zusammensetzung, wie im Anspruch 1 beansprucht, umfassend 0,01 bis 5 Gew.-% Terpineol.

3. Zusammensetzung, wie im Anspruch 1 oder 2 beansprucht, umfassend 0,1 bis 2 Gew.-% Thymol.

4. Zusammensetzung, wie in irgendeinem der Ansprüche 1 bis 3 beansprucht, umfassend 0,01 bis 2 Gew.-% Eugenol.

5. Zusammensetzung, wie in irgendeinem der vorhergehenden Ansprüche beansprucht, worin das Tensid ein kationisches, anionisches oder zwitterionisches Tensid ist.

6. Zusammensetzung, wie im Anspruch 5 beansprucht, worin das Tensid kationisch ist.

7. Zusammensetzung, wie im Anspruch 6 beansprucht, worin das kationische Tensid Benzalkoniumchlorid oder Cetyltrimethylammoniumbromid ist.

8. Zusammensetzung, wie in irgendeinem der vorhergehenden Ansprüche beansprucht, mit einem pH von 4 bis 7.

9. Zusammensetzung, wie in irgendeinem der vorhergehenden Ansprüche beansprucht, umfassend 0,5 bis 2 Gew.-% Elektrolyte.

10. Zusammensetzung, wie in irgendeinem der vorhergehenden Ansprüche beansprucht, zur Verwendung beim Desinfizieren externer Oberflächen des menschlichen oder tierischen Körpers, zum Desinfizieren von weichen und porösen Substraten, wie Textilien, oder zum Desinfizieren harter Oberflächen.

11. Nicht-therapeutisches Verfahren zum Desinfizieren einer Oberfläche, umfassend die Schritte:
(i) Auftragen einer Zusammensetzung, wie in irgendeinem der vorhergehenden Ansprüche 1 bis 10 beansprucht, auf eine gewünschte Oberfläche und
(ii) Entfernen der Zusammensetzung von der Oberfläche nach einer vorgegebenen Zeitdauer.

12. Nicht-therapeutisches Verfahren, wie im Anspruch 11 beansprucht, wobei die Zusammensetzung vor dem Auftrag auf die Oberfläche mit Wasser in einem Verhältnis von 1:1 bis 1:100 verdünnt wird.

## Revendications

1. Composition de micro-émulsion antimicrobienne huile-dans-eau comprenant :
(i) de 0,1 à 10 % en masse de tensioactif ;
(ii) de 0,05 à 10 % en masse d'actif d'huile essentielle comprenant du thymol, du terpinéol, de l'eugénol ou des mélanges de ceux-ci ;
(iii) de 0,1 à 5 % en masse de polyphénol ; et
(iv) de 80 à 98 % en masse d'eau ;
dans laquelle ledit polyphénol comprend au moins 50 % de catéchines et dans laquelle lesdites chatéchines comprennent au moins 25 % de gallate d'épigallocatéchine ;
et dans laquelle le polyphénol est extrait de *Camellia sinensis.*

2. Composition selon la revendication 1 comprenant de 0,01 à 5 % en masse de terpinéol.

3. Composition selon la revendication 1 ou 2 comprenant de 0,1 à 2 % en masse de thymol.

4. Composition selon l'une quelconque des revendications 1 à 3 comprenant de 0,01 à 2 % en masse d'eugénol.

5. Composition selon l'une quelconque des revendications précédentes, dans laquelle ledit tensioactif est un tensioactif cationique, anionique ou zwitterionique.

6. Composition selon la revendication 5, dans laquelle ledit tensioactif est cationique.

7. Composition selon la revendication 6, dans laquelle ledit tensioactif cationique est le chlorure de benzalkonium ou le bromure de cétyltriméthylammonium.

8. Composition selon l'une quelconque des revendications précédentes présentant un pH de 4 à 7.

9. Composition selon l'une quelconque des revendications précédentes comprenant de 0,5 à 2 % en masse d'électrolytes.

10. Composition selon l'une quelconque des revendications précédentes pour une utilisation dans la désinfection de surfaces externes de corps humain ou animal, pour la désinfection de substrats souples et poreux comme du textile ou pour la désinfection de surfaces dures.

11. Procédé non-thérapeutique de désinfection d'une surface comprenant les étapes consistant :
(i) à appliquer une composition selon l'une quelconque des revendications précédentes 1 à 10 sur une surface souhaitée ; et,
(ii) à éliminer la composition de ladite surface après une durée prédéterminée.

12. Procédé non-thérapeutique selon la revendication 11, dans lequel ladite composition est diluée avec de l'eau dans un rapport de 1:1 à 1:100 avant l'application sur ladite surface.
